# EUROPÄISCHE PATENTSCHRIFT

(11) **EP 2 804 655 B1**
(45) Veröffentlichungstag und Bekanntmachung des Hinweises auf die Patenterteilung: **19.06.2024**
(21) Anmeldenummer: 13706912.6
(22) Anmeldetag: 16.01.2013
(51) Int. Cl.: A61M 25/02

(54) **VORRICHTUNG ZUM ABDECKEN EINES KATHETERZUGANGS ZU EINEM MENSCHLICHEN ODER TIERISCHEN KÖRPER, INSBESONDERE EINES ZENTRAL-VENÖSEN KATHETERZUGANGS**
DEVICE FOR COVERING A CATHETER ACCESS TO A HUMAN OR ANIMAL BODY, IN PARTICULAR A CENTRAL VENOUS CATHETER ACCESS
DISPOSITIF POUR RECOUVRIR UN ACCÈS POUR CATHÉTER DANS UN CORPS HUMAIN OU ANIMAL, NOTAMMENT UN ACCÈS POUR CATHÉTER VEINEUX CENTRAL

(30) Priorität: 18.01.2012 DE 202012000408 U
(43) Veröffentlichungstag der Anmeldung: 26.11.2014
(73) Patentinhaber: PFM Medical AG, 50996 Köln (DE)
(72) Erfinder: KNOBLOCH, Helmut, 52372 Kreuzau (DE)
(74) Vertreter: Lippert Stachow Patentanwälte Rechtsanwälte Part.mbB
(86) Internationale Anmeldenummer: PCT/EP2013/000111
(87) Internationale Veröffentlichungsnummer: WO 2013/107634

(56) Entgegenhaltungen:
- EP-A1- 1 698 368
- WO-A1-97/21459
- US-A- 4 898 587
- US-A1- 2009 149 814

## Beschreibung

Die Erfindung betrifft eine Vorrichtung zum Abdecken eines Katheterzugangs zu einem menschlichen oder tierischen Körper, insbesondere eines zentral-venösen Katheterzugangs.

Ein zentralvenöser Katheter (ZVK), auch bezeichnet als zentraler Venenkatheter, Zentralvenenkatheter oder zentral-venöser Zugang, wird beispielsweise über eine Vene der oberen Körperhälfte in das Venensystem eingeführt, wobei das Ende des zentral-venösen Katheters in der oberen oder unteren Hohlvene vor dem rechten Vorhof des Herzens liegt. Alternativ kann ein zentral-venöser Katheter auch an einer anderen Stelle des menschlichen oder tierischen Körpers in das Venensystem eingeführt werden, wie z. B. über eine Beinvene (Vena Femoralis). Über den zentral-venösen Katheter können beispielsweise hoch konzentrierte Elektrolyt- und Nährstofflösungen in das Venensystem eingebracht werden. Weiterhin ermöglicht ein zentral-venöser Katheter eine Messung des zentral-venöses Druckes (ZVD) als Anhalt für das intravaskuläre Volumen (gleich Blutvolumen).

Ein zentral-venöser Katheter kann bis zu sechs Lumina umfassen, wobei üblicherweise zwei- oder dreilumige zentral-venöse Katheter eingesetzt werden. Über die einzelnen Lumen können beispielsweise parenterale Ernährung, Katecholamine und Antibiotika parallel in das Venensystem eingebracht werden, ohne die Gefahr von Inkompatibilitäten zwischen den einzelnen Substanzen.

Die Verwendung eines zentral-venösen Katheters kann zu einer Besiedelung, insbesondere der Eintrittsstelle des zentral-venösen Katheters in den menschlichen oder tierischen Körper, durch Bakterien oder Pilze führen. Durch die Bakterien oder Pilze können eine sogenannte Zentralvenenkatheter assoziierte Bakteriämie bzw. Fungämie oder eine Kathetersepsis (catheter related blood screen infection, CRBSI) verursachen, welche für den Patienten lebensbedrohlich sind.

Aus dem Stand der Technik ist es bekannt, den Katheterzugang mittels eines selbstklebenden Pflasters relativ zu der Eintrittsstelle in den menschlichen oder tierischen Körper zu fixieren und die Eintrittsstelle abzudecken. Weiterhin umfasst beispielsweise das von der Firma 3M Medica unter dem Namen "Tegaderm CHG" vertriebene selbstklebende Pflaster ein Gelpad mit einer anti-mikrobiellen Aktivität, wodurch eine Besiedlung durch Bakterien oder Pilze verhindert werden soll.

Das von der Firma Mediglobe vertriebene selbstklebende Pflaster "Glycosave Soft" umfasst ebenfalls ein anti-mikrobielles Gelpad im Bereich der Eintrittsstelle des Katheters, um eine Besiedelung durch Bakterien oder Pilze zu vermeiden.

Die aus dem Stand der Technik bekannten Vorrichtungen zum Abdecken eines Katheterzugangs zu einem menschlichen oder tierischen Körper, insbesondere eines zentral-venöses Katheterzugangs, haben jedoch den Nachteil, dass der Katheter im Bereich der Eintrittsstelle geknickt wird, wodurch sich der Querschnitt der Lumina innerhalb des Katheters verändert, was zu einer veränderten Durchflussrate führen kann.

Siehe, zum Beispiel, US 4 898 587 A, US 2009/149814 A1, WO 97/21459 A1 und EP 1 698 368 A1.

Der vorliegenden Erfindung liegt somit die Aufgabe zugrunde, eine Vorrichtung zum Abdecken eines Katheterzugangs zu einem menschlichen oder tierischen Körper (insbesondere eines zentral-venösen Katheterzugangs) bereitzustellen, welche ein Abknicken des Katheters im Bereich der Eintrittsstelle in den menschlichen oder tierischen Körper weitestgehend vermeidet.

Die Aufgabe wird gelöst durch die erfinderische Vorrichtung gemäß dem unabhängigen Anspruch 1 sowie der nachfolgenden abhängigen Ansprüche 2 - 13 zum Abdecken eines Katheterzugangs zu einem menschlichen oder tierischen Körper, insbesondere eines zentral-venösen Katheterzugangs, umfassend ein keilförmiges Führungselement, welches während der Verwendung zwischen dem Katheder des Katheterzugangs und der Oberfläche des menschlichen oder tierischen Körpers angeordnet ist, um den Katheter relativ zu einer Eintrittsstelle in dem menschlichen oder tierischen Körper zu führen; und ein selbstklebendes Abdeckelement zur Abdeckung und Fixierung des keilförmigen Führungselements und des Katheters im Bereich der Eintrittsstelle in den menschlichen oder tierischen Körper, welches bei der Verwendung derart auf die Oberfläche des menschlichen oder tierischen Körper geklebt wird, dass der Katheter und das keilförmige Führungselement wenigstens teilweise zwischen dem Abdeckelement und der Oberfläche des menschlichen oder tierischen Körpers angeordnet sind.

Das keilförmige Führungselement wird dabei so angeordnet, dass die spitzzulaufende Seite des keilförmigen Führungselements im Bereich der Eintrittsstelle des Katheters in dem menschlichen oder tierischen Körper angeordnet ist. Dadurch wird erreicht, dass der Katheter in einem spitzen Winkel auf die Eintrittsstelle in den menschlichen oder tierischen Körper verläuft und nach dem Eintritt in den menschlichen oder tierischen Körper ohne ein Abknicken an der Eintrittsstelle in das Venensystem weiter eingeführt werden kann. Das selbstklebende Abdeckelement dient dabei zur Vermeidung einer Besiedelung durch Bakterien oder Pilze und zur Fixierung des keilförmiges Führungselements und des Katheters im Bereich der Eintrittsstelle in den menschlichen oder tierischen Körper relativ zu dem menschlichen oder tierischen Körper.

Die erfinderische Vorrichtung umfasst weiterhin ein Fixierungselement, welches bei der Verwendung zwischen dem Katheter und dem selbstklebenden Abdeckelement angeordnet ist und zur Fixierung des Katheters relativ zu der Eintrittsstelle und dem selbstklebenden Abdeckelement ausgebildet ist. Der Katheter wird somit mittels des keilförmiges Führungselements und des weiteren Fixierungselements im Bereich der Eintrittsstelle in den menschlichen oder tierischen Körper fixiert und nachfolgend mit dem selbstklebenden Abdeckelement relativ zu dem menschlichen oder tierischen Körper fixiert und abgedeckt. Weiterhin hat das zusätzliche Fixierungselement den Vorteil, dass das selbstklebende Abdeckelement nicht direkt in Kontakt ist mit dem Katheter, was zu Komplikationen bei der Entfernung des selbstklebenden Abdeckelements von dem menschlichen oder tierischen Körper führen könnte, da der Katheter an dem selbstklebenden Abdeckelement bei dessen Entfernung von dem menschlichen oder tierischen Körper kleben bleiben könnte.

Das keilförmige Führungselement, sowie das Fixierungselement und optional das selbstklebende Abdeckelement sind gelartig ausgebildet. Dies hat den Vorteil, dass sich die einzelnen Elemente besser an die Kontur des Katheters und/oder des menschlichen oder tierischen Körpers anpassen können, wodurch der Katheter insbesondere im Bereich der Eintrittsstelle in den menschlichen oder tierischen Körper besser fixiert wird. Vorzugsweise wird diesbezüglich ein glycerinhaltiges Gel zur Herstellung verwendet.

In einer vorteilhaften Variante der Erfindung sind das selbstklebende Abdeckelement, das Fixierungselement und/oder das keilförmiges Führungselement zumindest teilweise transparent ausgebildet. Insbesondere sind die Elemente derart transparent ausgebildet, dass die Eintrittsstelle des Katheters in den menschlichen oder tierischen Körper ohne ein Entfernen der erfindungsgemäßen Vorrichtung einsehbar ist, so dass beispielsweise festgestellt werden kann, ob sich die Eintrittsstelle entzündet hat.

Gemäß einer zweckmäßigen Variante der Erfindung haben das selbstklebende Abdeckelement, das Fixierungselement und/oder das keilförmige Führungselement eine antimikrobielle, antibakterielle und/oder antimykotische Wirkung.

Nach einer weiteren vorteilhaften Variante der Erfindung sind das selbstklebende Abdeckelement, das Fixierungselement und/oder das keilförmige Führungselement feuchtigkeitsabsorbierend, wasserdicht und/oder atmungsaktiv. Feuchtigkeitsabsorbierende Elemente haben den Vorteil, dass diese eine eventuell aus der Eintrittsstelle des Katheters in den menschlichen oder tierischen Körper austretende Flüssigkeit aufnehmen können, so dass diese keinen negativen Einfluss auf die Eintrittsstelle in den menschlichen oder tierischen Körper beziehungsweise den menschlichen oder tierischen Körper im Bereich der Eintrittsstelle ausüben können. Vorzugsweise sind die feuchtigkeitsabsorbierenden Elemente derart ausgebildet, dass die absorbierte Flüssigkeit gespeichert wird und nicht wieder aus den feuchtigkeitsabsorbierenden Elementen austreten kann. Wasserdichte Elemente verhindern ein Eindringen von Flüssigkeiten, welche unter Umständen mit Bakterien belastet sind, in die Eintrittsstelle in den menschlichen oder tierischen Körper. Atmungsaktive Elemente ermöglich einen Wasserdampf- und Sauerstoffaustausch in den Bereichen, in welchen die erfindungsgemäße Vorrichtung auf die Oberfläche des menschlichen oder tierischen Körpers aufgebracht ist, wodurch eine Reizung der Oberfläche des menschlichen oder tierischen Körpers im Bereich der erfindungsgemäßen Vorrichtung weitestgehend vermieden werden kann. Zweckmäßigerweise ist das selbstklebende Abdeckelement wasserdicht und atmungsaktiv ausgebildet, während das Fixierungselement und das kleiförmiges Führungselement feuchtigkeitsabsorbierend ausgebildet sind.

In einer besonders vorteilhaften Variante der Erfindung sind das keilförmige Führungselement und/oder das Fixierungselement selbstklebend, insbesondere auf der bei der Verwendung dem menschlichen oder tierischen Körper zugewandten Oberfläche. Eine derartige Ausgestaltung hat den Vorteil, dass nach dem Einführen des Katheters in den menschlichen oder tierischen Körper das keilförmige Führungselement im Bereich der Eintrittsstelle des Katheters in den menschlichen oder tierischen Körper auf die Oberfläche des menschlichen oder tierischen Körpers geklebt werden kann, nachfolgend gegebenenfalls das weitere Fixierungselement relativ zu dem keilförmigen Führungselement und dem darauf angeordneten Katheter fixiert werden kann und nachfolgend das keilförmige Führungselement, der Katheter und das zusätzliche Fixierungselement mittels des selbstklebenden Abdeckelements relativ zu der Oberfläche des menschlichen oder tierischen Körpers fixiert werden können.

Zweckmäßigerweise sind die verwendeten Kleber latexfrei, vorzugsweise hypoallergen, beispielsweise ein Acrylatkleber. Dadurch wird eine besondere Verträglichkeit für den menschlichen oder tierischen Körper erzielt.

Zweckmäßigerweise bestehen das keilförmige Führungselement, das Fixierungselement und/oder das selbstklebende Abdeckelement aus Polyurethan, Polypropylen und/oder Polyethylen.

Nach einer weiteren Variante der erfindungsgemäßen Vorrichtung weist das keilförmige Führungselement einen Winkel zwischen 1 Grad und 30 Grad, vorzugsweise zwischen 3 Grad und 20 Grad und insbesondere zwischen 5 Grad und 10 Grad auf. Der Winkel bestimmt dabei die Steigung des keilförmigen Führungselements. Durch die genannten Winkel wird ein besonders fließender Übergang des Katheters in das Venensystem des menschlichen oder tierischen Körpers erzielt, so dass der Katheter im Bereich der Eintrittsstelle nicht derart geknickt wird, dass sich die Durchmesser der Lumina innerhalb des Katheters verändern.

In einer weiteren Variante der erfindungsgemäßen Vorrichtung weist das keilförmige Führungselement im Bereich der aufeinander zulaufenden Oberfläche eine Ausnehmung auf, so dass in dem Bereich zwei Vorsprünge entstehen. Vorzugsweise ist die Ausnehmung so ausgebildet, dass die Eintrittsstelle in den menschlichen oder tierischen Körper bei der Verwendung der Vorrichtung im Bereich der Ausnehmung angeordnet ist und die Vorsprünge die Eintrittsstelle seitlich zumindest teilweise umschließen. Dadurch wird eine bessere Fixierung des Katheters relativ zu der Eintrittsstelle in den menschlichen oder tierischen Körper erzielt. Beispielsweise ist die Ausnehmung dreieckig ausgebildet und die Spitze der dreieckigen Ausnehmung ist von der zulaufenden Seite des keilförmigen Führungselements entfernt angeordnet.

Zweckmäßigerweise sind das keilförmige Führungselement, das Fixierungselement und/oder das selbstklebende Abdeckelement an eine bestimmte Kathetergröße und/oder Katheterform angepasst, beispielsweise durch entsprechend der Größe oder Form des Katheters ausgebildete Vertiefungen zur Aufnahme der Kontur des Katheters.

Das Fixierungselement weist auf der bei der Verwendung dem menschlichen oder tierischen Körper zugewandten Seite Vertiefungen oder Ausnehmungen für die Eintrittsstelle, den Katheter und das keilförmige Führungselement auf. Da das Fixierungselement somit an die Konturen der umliegenden Elemente bzw. des Katheters und der Eintrittsstelle angepasst ist, wird eine bessere Fixierung des Katheters relativ zu der Eintrittsstelle in den menschlichen oder tierischen Körper erzielt.

Zweckmäßigerweise weist das selbstklebende Abdeckelement seitliche Flügel auf, welche den Katheter an der der Eintrittsstelle entfernt liegenden Seite seitlich umschließen, vorzugsweise in dem Bereich, wo eine Zuleitung an den Katheterzugang anschließbar ist. Dadurch kann das selbstklebende Abdeckelement auf dem keilförmigen Führungselement und dem gegebenenfalls vorhandenen zusätzlichen Fixierungselement angeordnet werden, selbst wenn der Katheter in den menschlichen oder tierischen Körper eingeführt ist und an dem Katheter bereits eine Zuleitung angeschlossen ist. Weiterhin ist das selbstklebende Abdeckelement zweckmäßigerweise so ausgebildet, dass das selbstklebende Abdeckelement bei der Verwendung eines zusätzlichen Fixierungselements nicht in direkten Kontakt mit dem Katheter ist.

Aufgrund der Fixierung des Katheterzugangs relativ zu dem menschlichen oder tierischen Körper und der Abdeckung der Eintrittsstelle des Katheters in den menschlichen oder tierischen Körper ist die erfindungsgemäße Vorrichtung für Langzeitanwendungen geeignet, insbesondere für eine Tragezeit bis zu zehn Tage, vorzugsweise 8 Tage.

Die erfindungsgemäße Vorrichtung ist neben zentral-venösen Katheterzugängen auch für peripher eingeführte zentrale Katheter, arterielle Katheter, Port-Katheter-Systeme oder Epidural-Katheter einsetzbar.

Nachfolgend wird die Erfindung anhand von in den Figuren dargestellten Ausführungsbeispielen näher erläutert. Es zeigen:
- Figur 1: Eine seitliche Schnittdarstellung einer beispielhaften Vorrichtung,
- Figur 2a: ein keilförmiges Führungselement zur Verwendung mit einer beispielhaften Vorrichtung,
- Figur 2b: ein weiteres keilförmiges Führungselement zur Verwendung mit einer beispielhaften Vorrichtung,
- Figur 3: ein selbstklebendes Abdeckelement zur Verwendung mit einer beispielhaften Vorrichtung,
- Figur 4: eine perspektivische Darstellung einer erfindungsgemäßen Vorrichtung aus Richtung des tierischen oder menschlichen Körpers,
- Figur 5: eine Detailansicht des keilförmigen Führungselements aus Figur 4, und
- Figur 6: eine Detailansicht des Fixierungselements aus Figur 4.

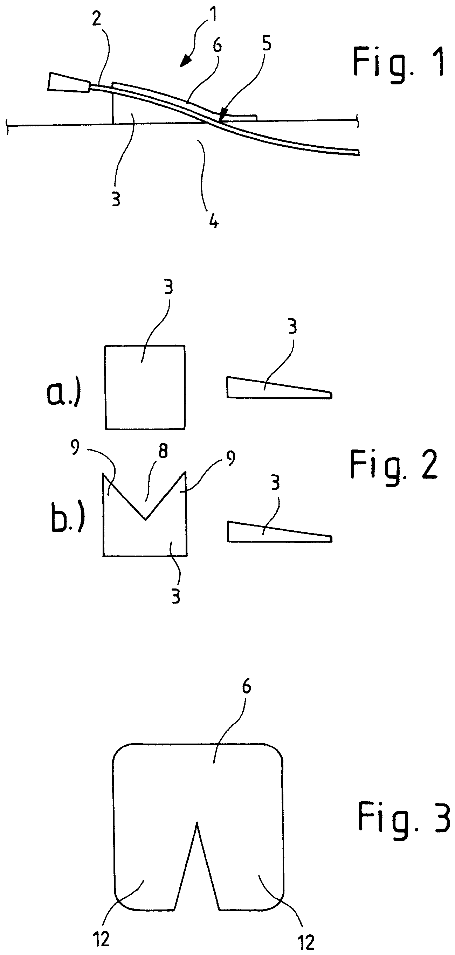

Figur 1 zeigt eine Vorrichtung 1 zum Abdecken eines Katheterzugangs zu einem menschlichen oder tierischen Körper 4, insbesondere eines zentral-venösen Katheterzugangs. Die Vorrichtung 1 umfasst ein keilförmiges Führungselement 3, welches während der Verwendung zwischen dem Katheter 2 des Katheterzugangs und der Oberfläche des menschlichen oder tierischen Körpers 4 angeordnet ist, um den Katheter 2 relativ zu einer Eintrittsstelle 5 in den menschlichen oder tierischen Körper 4 zu führen. Die Vorrichtung 1 umfasst weiterhin ein selbstklebendes Abdeckelement 6 zur Abdeckung und Fixierung des keilförmiges Führungselements 3 und des Katheters 2 im Bereich der Eintrittsstelle 5 in den menschlichen oder tierischen Körper 4. Das selbstklebende Abdeckelement 6 ist bei der Verwendung derart auf die Oberfläche des menschlichen oder tierischen Körpers 4 geklebt, dass der Katheter 2 und das keilförmiges Führungselement 3 wenigstens teilweise zwischen dem Abdeckelement 6 und der Oberfläche des menschlichen oder tierischen Körpers 4 angeordnet sind.

Somit werden erfindungsgemäß mittels der Vorrichtung 1 zum Abdecken eines Katheterzugangs zu einem menschlichen oder tierischen Körper 4, insbesondere eines zentral-venösen Katheterzugangs, der Katheter 2 im Bereich der Eintrittsstelle 5 in den menschlichen oder tierischen Körper 4 derart geführt, dass der Katheter 2 nicht abgeknickt wird, insbesondere derart dass es zu einer Veränderung der Durchmesser der Lumina innerhalb des Katheters 2 kommt. Weiterhin wird die Eintrittsstelle 5 in den menschlichen oder tierischen Körper 4 durch das selbstklebende Abdeckelement 6 vor einer Besiedlung durch Bakterien oder Pilz geschützt.

Das keilförmige Führungselement 3 aus Figur 1 ist gelartig ausgebildet, damit es sich an die Oberfläche des menschlichen oder tierischen Körpers 4 anpassen kann. Eine gelartige Ausbildung des keilförmigen Führungselements 3 hat weiterhin den Vorteil, dass sich das keilförmige Führungselement 3 an die Form des Katheters 2 anpassen kann. Das selbstklebende Abdeckelement 6 aus Figur 1 ist erfindungsgemäß möglichst flexibel ausgebildet, damit es sich an die Kontur des keilförmigen Führungselements 3, des Katheters 2 und der Oberfläche des menschlichen oder tierischen Körpers 4 anpassen kann.

Das selbstklebende Abdeckelement 6 und das keilförmige Führungselement 3 aus Figur 1 sind zumindest teilweise transparent ausgebildet, insbesondere im Bereich der Eintrittsstelle 5 des Katheters 2 in den menschlichen oder tierischen Körper 4. Somit kann die Eintrittsstelle 5 des Katheters 2 in den menschlichen oder tierischen Körper 4 während der gesamten Anwendungszeit des Katheters 2 auf eventuell auftretende Anomalien, wie beispielsweise Entzündungen untersucht werden, ohne dass die selbstklebende Abdeckung 6 und/oder das keilförmige Führungselement 3 entfernt werden müssten.

Das selbstklebende Abdeckelement 6 und das keilförmige Führungselement 3 aus Figur 1 haben eine antimikrobielle, antibakterielle und antimykotische Wirkung, um eine Besiedelung der Eintrittsstelle 5 in den menschlichen oder tierischen Körper 4 durch Bakterien oder Pilze zu verhindern. Dadurch wird das Risiko einer Zentralvenenkatheter assoziierten Bakteriämie bzw. Fugämie oder einer Kathetersepsis deutlich reduziert.

Das keilförmige Führungselement 3 aus Figur 1 ist feuchtigkeitsabsorbierend ausgebildet, um eventuell aus der Eintrittsstelle 5 des Katheters 2 in den menschlichen oder tierischen Körpers 4 austretende Flüssigkeiten zu absorbieren. Weiterhin ist das selbstklebende Abdeckelement 6 aus Figur 1 wasserdicht und atmungsaktiv ausgebildet, wodurch einerseits ein Eintreten von Flüssigkeiten vermieden wird und andererseits ein Wasserdampf- und Sauerstoffaustausch von dem menschlichen oder tierischen Körper 4 in Richtung Umgebung ermöglicht wird.

Zweckmäßigerweise ist das keilförmige Führungselement 3 aus Figur 1 selbstklebend ausgebildet, insbesondere auf der bei der Verwendung dem menschlichen oder tierischen Körper 4 zugewandten Oberfläche. Damit kann das keilförmige Führungselement 3 relativ zu der Eintrittsstelle 5 des Katheters 2 in den menschlichen oder tierischen Körper 4 fixiert werden, bevor es durch das selbstklebende Abdeckelement 6 fixiert wird.

Sämtliche bei der erfindungsgemäßen Vorrichtung 1 genannten Kleber sind latexfrei und hypoallergen, um die Oberfläche des menschlichen oder tierischen Körpers 4 zu schonen.

Das keilförmige Führungselement 3 und das selbstklebende Abdeckelement 6 aus Figur 1 bestehen beispielsweise aus Polyurethan.

Wie insbesondere den Figuren 2a und 2b entnommen werden kann, weist das keilförmige Führungselement 3 einen Winkel zwischen 1 Grad und 30 Grad, vorzugsweise zwischen 3 Grad und 20 Grad und insbesondere zwischen 5 Grad und 10 Grad auf. Wobei der Winkel die Steigung des keilförmigen Führungselements 3 beschreibt.

In der Ausführungsform gemäß Figur 2b weist das keilförmige Führungselement 3 im Bereich der aufeinander zulaufenden Oberflächen eine Ausnehmung 8 auf, so dass in diesem Bereich zwei Vorsprünge 9 entstehen. Das keilförmige Führungselement 3 gemäß Figur 2b wird derart auf der Oberfläche des menschlichen oder tierischen Körpers 4 angeordnet, dass sich die Eintrittsstelle 5 des Katheters 2 in den menschlichen oder tierischen Körper 4 im Bereich der Ausnehmung 8 befindet und die zwei Vorsprünge 9 die Eintrittsstelle 5 des Katheters 2 in den menschlichen oder tierischen Körper 4 seitlich zumindest teilweise umschließen.

Die Ausnehmung 8 in dem keilförmigen Führungselement 3 aus Figur 2b ist dreieckig ausbildet, wobei die Spitze der dreieckigen Ausnehmung 8 von der zulaufenden Seite des keilförmigen Führungselements 3 entfernt angeordnet ist.

Figur 3 zeigt eine Detailansicht eines selbstklebenden Abdeckelements 6 zur Abdeckung und Fixierung des keilförmigen Führungselements 3 und des Katheters 2 im Bereich der Eintrittsstelle 5 in den menschlichen oder tierischen Körper 4. Das selbstklebende Abdeckelement 6 aus Figur 3 wird bei der Verwendung derart auf die Oberfläche des menschlichen oder tierischen Körpers 4 geklebt, dass der Katheter 2 und das keilförmige Führungselement 3 wenigstens teilweise zwischen dem Abdeckelement 6 und der Oberfläche des menschlichen der tierischen Körpers 4 angeordnet sind. Das selbstklebende Abdeckelement 6 aus Figur 3 weist zwei seitliche Flügel 12 auf, welche den Katheter 2 an der der Eintrittsstelle 5 entfernt liegenden Seite seitlich umschließen. Dadurch kann das selbstklebende Abdeckelement 6 auf dem keilförmigen Führungselement 3 und dem Katheter 2 angeordnet werden, selbst wenn der Katheter 2 in dem menschlichen oder tierischen Körper 4 eingeführt ist und an dem Katheter 2 bereits eine Zuleitung angeschlossen ist.

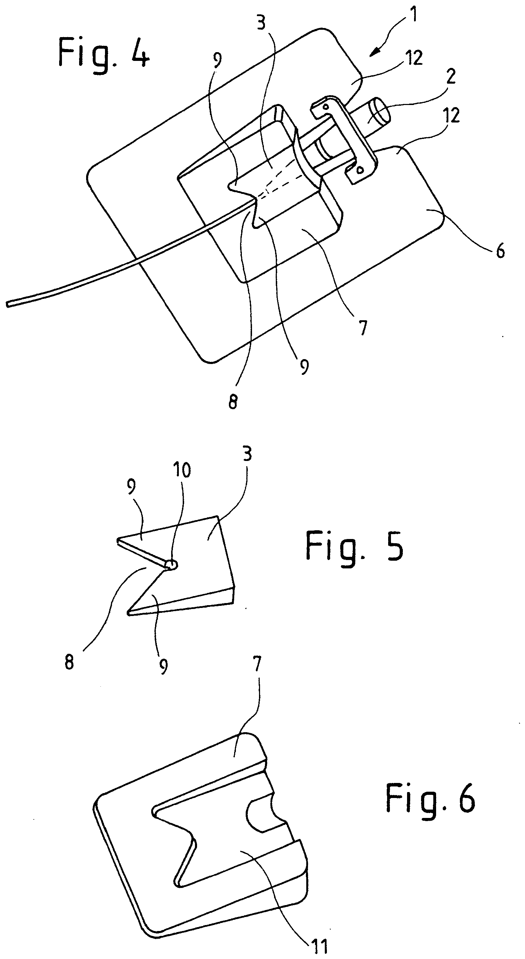

In Figur 4 ist eine erfindungsgemäße Vorrichtung 1 zum Abdecken eines Katheterzugangs zu einem menschlichen oder tierischen Körper 4, insbesondere eines zentral-venösen Katheterzugangs, dargestellt. Die Vorrichtung 1 aus Figur 4 umfasst ein keilförmiges Führungselement 3, welches während der Verwendung zwischen dem Katheter 2 des Katheterzugangs und der Oberfläche des menschlichen oder tierischen Körpers 4 angeordnet ist, um den Katheter 3 relativ zu einer Eintrittsstelle 5 in den menschlichen oder tierischen Körper 4 zu führen.

Die Vorrichtung 1 aus Figur 4 umfasst weiterhin ein Fixierungselement 7, welches bei der Verwendung zwischen dem Katheter 2 und einem selbstklebenden Abdeckelement 6 angeordnet ist und zur Fixierung des Katheters 2 relativ zu der Eintrittsstelle 5 und dem selbstklebenden Abdeckelement 6 ausgebildet ist.

Das selbstklebende Abdeckelement 6 der Vorrichtung 1 gemäß Figur 4 dient zur Abdeckung und Fixierung des keilförmigen Führungselements 3, des Katheters 2 und des Fixierungselements 7 insbesondere im Bereich der Eintrittsstelle 5 in den menschlichen oder tierischen Körper 4. Das selbstklebende Abdeckelement 6 wird bei der Verwendung derart auf die Oberfläche des menschlichen oder tierischen Körpers 4 geklebt, dass zwischen Katheter 2 und der Oberfläche des menschlichen oder tierischen Körpers 4 das keilförmige Führungselement 3 angeordnet ist und zwischen dem Katheter 2 und dem selbstklebendem Abdeckelement 6 das Fixierungselement 7 angeordnet ist.

Das keilförmige Führungselement 3 und das Fixierungselement 7 aus Figur 4 sind gelartig ausgebildet, damit diese sich besser an die Kontur des menschlichen der tierischen Körpers 4 bzw. des Katheters 2 anpassen können.

Das selbstklebende Abdeckelement 6, das Fixierungselement 7 und das keilförmige Führungselement 3 aus Figur 4 sind zumindest im Bereich der Eintrittsstelle 5 des Katheter 2 in den menschlichen oder tierischen Körper 4 transparent ausgebildet, um die Eintrittsstelle 5 des Katheters 2 in den menschlichen oder tierischen Körper während der Verwendung der erfindungsgemäßen Vorrichtung 1 beobachten zu können.

Zweckmäßigerweise haben das selbstklebende Abdeckelement 6, das Fixierungselement 7 und das keilförmige Führungselement 3 eine antimikrobielle, antibakterielle und antimykotische Wirkung, um die Eintrittsstelle 5 des Katheters 2 in den menschlichen oder tierischen Körper 4 vor einer Besiedelung durch Bakterien und/oder Pilze zu schützen.

Das selbstklebende Abdeckelement 6 aus Figur 4 ist wasserdicht und atmungsaktiv ausgebildet, um einen Eintritt von Flüssigkeiten in die Eintrittsstelle des Katheters 2 in den menschlichen oder tierischen Körper 4 zu vermeiden und gleichzeitig die Oberfläche des menschlichen oder tierischen Körper 4 im Bereich der Eintrittsstelle 5 des Katheters 2 in den menschlichen oder tierischen Körper 4 zu schonen.

Das Fixierungselement 7 und das keilförmige Führungselement 3 aus Figur 4 sind zweckmäßigerweise feuchtigkeitsabsorbierend ausgebildet, um eventuell aus der Eintrittsstelle 5 des Katheters 2 in den menschlichen oder tierischen Körper 4 austretende Flüssigkeiten zu absorbieren.

Weiterhin sind das keilförmige Führungselement 3 und das Fixierungselement 7 selbstklebend ausgebildet, insbesondere auf der bei der Verwendung dem menschlichen oder tierischen Körper 4 zugewandten Oberfläche. Dadurch können das keilförmige Führungselement 3 und das Fixierungselement 7 relativ zu der Oberfläche des menschlichen oder tierischen Körpers 4 fixiert werden bevor das selbstklebende Abdeckelement 6 auf das keilförmige Führungselement 3 bzw. das Fixierungselement 7 aufgebracht wird.

Die verwendeten Kleber der erfindungsgemäßen Vorrichtung 1 aus Figur 4 sind latexfrei und hypoallergen, um die Oberfläche des menschlichen oder tierischen Körpers 4 zu schonen.

Das keilförmige Führungselement 3, das Fixierungselement 7 und das selbstklebende Abdeckelement bestehen aus Polyurethan.

Wie insbesondere der Detailansicht des keilförmigen Führungselements 3 aus Figur 5 entnommen werden kann, weist das keilförmige Führungselement 3 einen Winkel zwischen 1 Grad und 30 Grad, vorzugsweise zwischen 3 Grad und 20 Grad und insbesondere zwischen 5 Grad und 10 Grad auf. Der Winkel gibt dabei die Steigung des keilförmigen Führungselements 3 an.

Das keilförmige Führungselement 3 weist im Bereich der aufeinander zulaufenden Oberflächen eine Ausnehmung 8 auf, so dass in dem Bereich zwei Vorsprünge 9 entstehen, wie in Figur 5 dargestellt. Die Ausnehmung 8 ist so ausgebildet, dass die Eintrittsstelle 5 in den menschlichen oder tierischen Körper 4 bei der Verwendung der Vorrichtung 1 aus Figur 4 im Bereich der Ausnehmung 8 angeordnet ist und die Vorsprünge 9 die Eintrittsstelle 5 seitlich zumindest teilweise umschließen. Dadurch wird eine bessere Fixierung des Katheters 2 relativ zu der Eintrittsstelle 5 in den menschlichen oder tierischen Körper 4 erzielt. Die Ausnehmung 8 ist dreieckig ausgebildet, wobei die Spitze der dreieckigen Ausnehmung 8 von der zulaufenden Seite des keilförmigen Führungselements 3 entfernt angeordnet ist.

Das keilförmige Führungselement 3 und das Fixierungselement 7 sind an eine bestimme Kathetergröße und/oder Katheterform angepasst, beispielsweise durch entsprechend der Größe oder der Form des Katheters 2 ausgebildete Vertiefungen 10, 11 zur Aufnahme der Kontur des Katheters 2.

Insbesondere weist das Fixierungselement 7 auf der bei der Verwendung im menschlichen oder tierischen Körper 4 zugewandten Seite Vertiefungen oder Ausnehmungen 11 für die Eintrittsstelle 5, den Katheter 2 und/oder das keilförmige Führungselement 3 auf, wie der Detailansicht aus Figur 6 entnommen werden kann. Das in Figur 6 dargestellte Fixierungselement 7 ist wie das Führungselement 3 keilförmig. Alternativ kann das Fixierungselement 7 auch eine flache Form aufweisen.

Das selbstklebende Abdeckelement 6 aus Figur 4 weist seitliche Flügel 12 auf, welche den Katheter 2 an der der Eintrittsstelle 5 entfernt liegenden Seite seitlich umschließen, vorzugsweise in dem Bereich, wo eine Zuleitung an den Katheter 2 anschließbar ist. Das selbstklebende Abdeckelement 6 aus Figur 4 entspricht somit dem in Figur 3 dargestellten selbstklebenden Abdeckelement 6. Durch eine derartige Ausgestaltung kann das selbstklebende Abdeckelement 6 auf dem keilförmigen Führungselement 3 und dem zusätzlichen Fixierungselement 7 angeordnet werden, selbst wenn der Katheter 2 in den menschlichen oder tierischen Körper 4 eingeführt ist und an dem Katheter 2 bereits eine Zuleitung angeschlossen ist. Weiterhin ist das selbstklebende Abdeckelement 6 so ausgebildet, dass das selbstklebende Abdeckelement 6 bei der Verwendung eines zusätzlichen Fixierungselements 7 nicht in direktem Kontakt mit dem Katheter 2 ist.

Aufgrund der Fixierung des Katheterzugangs relativ zu dem menschlichen oder tierischen Körper 4 und der Abdeckung der Eintrittsstelle 5 des Katheters 2 in den menschlichen oder tierischen Körper 4 ist die erfindungsgemäße Vorrichtung 1 für Langzeitanwendungen geeignet, insbesondere für eine Tragezeit bis zu zehn Tage.

Weiterhin ist die erfindungsgemäße Vorrichtung 1 neben zentral-venösen Katheterzugängen auch für peripher eingeführte zentrale Katheter, arterielle Katheter, Port-Katheter-Systeme oder Epidural-Katheter einsetzbar.

### Bezugszeichenliste

- 1: Vorrichtung
- 2: Katheter
- 3: keilförmiges Führungselement
- 4: menschlicher oder tierischer Körper
- 5: Eintrittsstelle
- 6: selbstklebendes Abdeckelement
- 7: Fixierungselement
- 8: Ausnehmung im keilförmigen Führungselement
- 9: Vorsprung des keilförmiges Führungselements
- 10: Vertiefung für Kontur des Katheters
- 11: Vertiefung oder Ausnehmung im Fixierungselement
- 12: Flügel

## Patentansprüche

1. Vorrichtung (1) zum Abdecken eines Katheterzugangs zu einem menschlichen oder tierischen Körper (4), insbesondere eines zentral-venösen Katheterzugangs, umfassend:
ein keilförmiges, gelartiges Führungselement (3), welches während der Verwendung zwischen dem Katheter (2) des Katheterzugangs und der Oberfläche des menschlichen oder tierischen Körpers (4) angeordnet ist, um den Katheter (2) relativ zu einer Eintrittsstelle (5) in den menschlichen oder tierischen Körper (4) zu führen;
ein flexibles_selbstklebendes Abdeckelement (6) zur Abdeckung und Fixierung des keilförmigen Führungselements (3) und des Katheters (2) im Bereich der Eintrittsstelle (5) in den menschlichen oder tierischen Körper (4), welches bei der Verwendung derart auf die Oberfläche des menschlichen oder tierischen Körpers (4) geklebt wird, dass der Katheter (2) und das keilförmige Führungselement (3) wenigstens teilweise zwischen dem Abdeckelement (6) und der Oberfläche des menschlichen oder tierischen Körpers (4) angeordnet sind; und
ein gelartiges Fixierungselement (7), welches bei der Verwendung zwischen dem Katheter (2) und dem selbstklebenden Abdeckelement (6) angeordnet ist und zur Fixierung des Katheters (2) relativ zu der Eintrittsstelle (5) und dem selbstklebenden Abdeckelement (6) ausgebildet ist;
wobei das Fixierungselement (7) auf der bei der Verwendung dem menschlichen oder tierischen Körper (4) zugewandten Seite Vertiefungen oder Ausnehmungen (11) für die Eintrittsstelle (5), den Katheter (2) und das keilförmige Führungselement (3) aufweist.

2. Vorrichtung (1) nach Anspruch 1, wobei das selbstklebende Abdeckelement (6), das Fixierungselement (7) und/oder das keilförmige Führungselement (3) zumindest teilweise transparent ausgebildet sind.

3. Vorrichtung (1) nach einem der Ansprüche 1 bis 2, wobei das selbstklebende Abdeckelement (6), das Fixierungselement (7) und/oder das keilförmige Führungselement (3) eine antimikrobielle, antibakterielle und/oder antimykotische Wirkung haben.

4. Vorrichtung (1) nach einem der Ansprüche 1 bis 3, wobei das selbstklebende Abdeckelement (6), das Fixierungselement (7) und/oder das keilförmige Führungselement (3) feuchtigkeitsabsorbierend, wasserdicht und/oder atmungsaktiv sind.

5. Vorrichtung (1) nach einem der Ansprüche 1 bis 4, wobei das keilförmige Führungselement (3) und/oder das Fixierungselement (7) selbstklebend sind, insbesondere auf der bei der Verwendung dem menschlichen oder tierischen Körper (4) zugewandten Oberfläche.

6. Vorrichtung (1) nach einem der Ansprüche 1 bis 5, wobei die verwendeten Kleber latexfrei sind, vorzugsweise hypoallergen.

7. Vorrichtung (1) nach einem der Ansprüche 1 bis 6, wobei das keilförmige Führungselement (3), das Fixierungselement (7) und/oder das selbstklebende Abdeckelement (6) aus Polyurethan, Polypropylen und/oder Polyethylen bestehen.

8. Vorrichtung (1) nach einem der Ansprüche 1 bis 7, wobei das keilförmige Führungselement (3) einen Winkel zwischen 1 Grad und 30 Grad, vorzugsweise zwischen 3 Grad und 20 Grad und insbesondere zwischen 5 Grad und 10 Grad aufweist.

9. Vorrichtung (1) nach einem der Ansprüche 1 bis 8, wobei das keilförmige Führungselement (3) im Bereich der aufeinander zulaufenden Oberflächen eine Ausnehmung (8) aufweist, so dass in dem Bereich zwei Vorsprünge (9) entstehen.

10. Vorrichtung (1) nach Anspruch 9, wobei die Ausnehmung (8) so ausgebildet ist, dass die Eintrittsstelle (5) in den menschlichen oder tierischen Körper (4) bei der Verwendung der Vorrichtung (1) im Bereich der Ausnehmung (8) angeordnet ist und die Vorsprünge (9) die Eintrittsstelle (5) seitlich zumindest teilweise umschließen.

11. Vorrichtung (1) nach Anspruch 8 oder 9, wobei die Ausnehmung (8) dreieckig ausgebildet ist und die Spitze der dreieckigen Ausnehmung (8) von der zulaufenden Seite des keilförmigen Führungselements (3) entfernt angeordnet ist.

12. Vorrichtung (1) nach einem der Ansprüche 1 bis 11, wobei das keilförmige Führungselement (3), das Fixierungselement (7) und/oder das selbstklebende Abdeckelement (6) an eine bestimmte Kathetergröße und/oder Katheterform angepasst sind, beispielsweise durch entsprechend der Größe oder Form des Katheters (2) ausgebildete Vertiefungen (10,11) zur Aufnahme der Kontur des Katheters (2).

13. Vorrichtung (1) nach einem der Ansprüche 1 bis 12, wobei das selbstklebende Abdeckelement (6) seitliche Flügel (12) aufweist, welche den Katheter (2) an der der Eintrittsstelle (5) entfernt liegenden Seite seitlich umschließen, vorzugsweise in dem Bereich, wo eine Zuleitung an den Katheter (2) anschließbar ist.

## Claims

1. A device (1) for covering a catheter access of a catheter to a human or animal body (4), particularly a central-venous catheter access, comprising:
a wedge-shaped, gel-like guiding element (3) to be arranged between the catheter (2) of the catheter access and the surface of the human or animal body (4), configured to guide the catheter (2) relative to an entry point (5) into the human or animal body (4);
a flexible, self-adhesive covering element (6) which covers and fixes the wedge-shaped guiding element (3) and the catheter (2) in an area of the entry point (5) into the human or animal body;
wherein the self-adhesive covering element (6) is configured to be adhesively affixed directly to the surface of the human or animal body (4) that the catheter (2) and the wedge-shaped guiding element (3) are configured to be at least partially arranged between the covering element (6) and the surface of the human or animal body (4); and
a gel-like fixing element (7) configured to be arranged between the catheter (2) and the self-adhesive covering element (6) and configured such that the catheter (2) is fixed relative to the entry point (5) and the self-adhesive covering element (6);
wherein the fixing element (7) has recesses or cut-outs for the entry point (5), the catheter (2) and the wedge-shaped guiding element (3) on the side facing the human or animal body during use.

2. The device (1) according to claim 1, wherein the self-adhesive covering element (6), the fixing element (7) and/or the wedge-shaped guiding element (3) are at least partially transparent.

3. The device (1) according to any one of claims 1 to 2, wherein the self-adhesive covering element (6), the fixing element (7) and/or the wedge-shaped guiding element (3) have an anti-microbial, anti-bacterial and/or anti-fungal effect.

4. The device (1) according to any one of claims 1 to 3, wherein the self- adhesive covering element (6), the fixing element (7) and/or the wedge-shaped guiding element (3) are moisture absorbing, waterproof and breathable.

5. The device (1) according to any one of claims 1 to 4, wherein the wedge- shaped guiding element (3) and/or the fixing-element (7) is self-adhesive, in particular on the surfaces facing towards the human or animal body during use.

6. The device (1) according to any of claims 1 to 5, wherein the adhesive is latex-free, preferably hypoallergenic.

7. The device (1) according to any one of claims 1 to 6, wherein the wedge-shaped guiding element (3), the fixing element (7) and/or the self-adhesive covering element (6) are formed of polyurethane, polypropylene and/or polyethylene.

8. The device (1) according to any one of claims 1 to 7, wherein the wedge-shaped guiding element (3) has an angle between 1 degree and 30 degrees, preferably between 3 and 20 degrees and in particular between 5 and 10 degrees.

9. The device (1) according to any one of claims 1 to 8, wherein the wedge-shaped guiding element (3) has a recess (8) at the tapered end, and so the recess (8) is between two lateral protrusions (9).

10. The device (1) according to claim 9, wherein the recess (8) is built such that the entry point (5) into the human or animal body (4) is arranged in the region of the recess (8) when the device(1) is used and the protrusions (9) at least partially enclose the entry point (5) laterally.

11. The device (1) according to claim 8 or 9, wherein the recess (8) is triangular in shape and the tip of the triangular recess (8) is arranged at a distance from the tapered end of the wedge-shaped guiding element (3).

12. The device (1) according to any one of claims 1 to 11 wherein the wedge-shaped guiding element (3), the fixing element (7) and/or the self-adhesive covering element (6) are adapted to a particular catheter size and/or catheter profile, for example by cavities corresponding to the size or the profile of the catheter (2) for receiving an outline of the catheter (2).

13. The device (1) according to any one of claims 1 to 12, wherein the self-adhesive covering element (6) has lateral wings (12) which laterally enclose the catheter (2) at an end opposite to the entry point (5), preferably in an area where a hose can be connected to the catheter (2).

## Revendications

1. Un dispositif (1) de recouvrement d'accès d'un cathéter à un corps humain ou animal (4), en particulier d'accès d'un cathéter veineux central, comprenant :
un élément de guidage de type gel cunéiforme (3) à agencer pendant l'utilisation entre le cathéter (2) de l'accès du cathéter et la surface du corps humain ou animal (4), conçu pour guider le cathéter (2) par rapport à un point d'entrée (5) dans le corps humain ou animal (4) ;
un élément de recouvrement auto-adhésif flexible (6), qui recouvre et fixe l'élément de guidage cunéiforme (3) et le cathéter (2) dans une zone du point d'entrée (5) dans le corps humain ou animal pendant l'utilisation, est fixé de manière adhésive directement sur la surface du corps humain ou animal (4) de telle sorte que le cathéter (2) et l'élément de guidage cunéiforme (3) sont au moins partiellement agencés entre l'élément de recouvrement (6) et la surface du corps humain ou animal (4) ; et
un élément de fixation de type gel (7) conçu pour être agencé pendant l'utilisation entre le cathéter (2) et l'élément de recouvrement auto-adhésif (6) et conçu de telle sorte que le cathéter (2) est fixé par rapport au point d'entrée (5) et à l'élément de recouvrement auto-adhésif (6) ;
dans lequel l'élément de fixation (7) a des évidements ou des découpes pour le point d'entrée (5), le cathéter (2) et l'élément de guidage cunéiforme (3) sur le côté faisant face au corps humain ou animal pendant l'utilisation.

2. Le dispositif (1) selon la revendication 1, dans lequel l'élément de recouvrement auto-adhésif (6), l'élément de fixation (7) et/ou l'élément de guidage cunéiforme (3) sont au moins partiellement transparents.

3. Le dispositif (1) selon l'une quelconque des revendications 1 à 2, dans lequel l'élément de recouvrement auto-adhésif (6), l'élément de fixation (7) et/ou l'élément de guidage cunéiforme (3) ont un effet antimicrobien, antibactérien et/ou antifongique.

4. Le dispositif (1) selon l'une quelconque des revendications 1 à 3, dans lequel l'élément de recouvrement auto-adhésif (6), l'élément de fixation (7) et/ou l'élément de guidage cunéiforme (3) sont absorbeurs d'humidité, étanches à l'eau et/ou perméables à l'air.

5. Le dispositif (1) selon l'une quelconque des revendications 1 à 4, dans lequel l'élément de guidage cunéiforme (3) et/ou l'élément de fixation (7) sont auto-adhésifs, en particulier sur la surface orientée vers le corps humain ou animal pendant l'utilisation.

6. Le dispositif (1) selon l'une quelconque des revendications 1 à 5, dans lequel l'adhésif est exempt de latex, de préférence hypoallergénique.

7. Le dispositif (1) selon l'une quelconque des revendications 1 à 6, dans lequel l'élément de guidage cunéiforme (3), l'élément de fixation (7) et/ou l'élément de recouvrement auto-adhésif (6) sont formés de polyuréthane, de polypropylène et/ou de polyéthylène.

8. Le dispositif (1) selon l'une quelconque des revendications 1 à 7, dans lequel l'élément de guidage cunéiforme (3) a un angle compris entre 1 degré et 30 degrés, de préférence compris entre 3 et 20 degrés et en particulier compris entre 5 et 10 degrés.

9. Le dispositif (1) selon l'une quelconque des revendications 1 à 8, dans lequel l'élément de guidage cunéiforme (3) a un évidement (8) au niveau de l'extrémité effilée, et ainsi l'évidement (8) se situe entre deux protubérances latérales (9).

10. Le dispositif (1) selon la revendication 9, dans lequel l'évidement (8) est élaboré de telle sorte que le point d'entrée (5) dans le corps humain ou animal (4) est agencé dans la région de l'évidement (8) lorsque le dispositif (1) est utilisé et que les protubérances (9) entourent au moins partiellement le point d'entrée (5) de manière latérale.

11. Le dispositif (1) selon la revendication 8 ou 9, dans lequel l'évidement (8) est de forme triangulaire et la pointe de l'évidement (8) triangulaire est agencée à une distance de l'extrémité effilée de l'élément de guidage cunéiforme (3).

12. Le dispositif (1) selon l'une quelconque des revendications 1 à 11, dans lequel l'élément de guidage cunéiforme (3), l'élément de fixation (7) et/ou l'élément de recouvrement auto-adhésif (6) sont adaptés à une taille de cathéter particulière et/ou à un profil de cathéter, par exemple par des cavités correspondant à la taille ou au profil du cathéter (2) pour la réception d'un contour du cathéter (2).

13. Le dispositif (1) selon l'une quelconque des revendications 1 à 12, dans lequel l'élément de recouvrement auto-adhésif (6) a des ailes latérales (12) qui entourent de manière latérale le cathéter (2) au niveau d'une extrémité opposée au point d'entrée (5), de préférence dans une zone où un tuyau peut être relié au cathéter (2).
